# EUROPEAN PATENT SPECIFICATION

(11) **EP 0 799 008 B1**
(45) Date of publication and mention of the grant of the patent: **20.03.2002**
(21) Application number: 95944095.9
(22) Date of filing: 14.12.1995
(51) Int. Cl.: A61F 13/62

(54) **FASTENING TAB**
BEFESTIGUNGSLASCHE
LANGUETTE DE FIXATION

(30) Priority: 22.12.1994 US 362394
(43) Date of publication of application: 08.10.1997
(73) Proprietor: KIMBERLY-CLARK WORLDWIDE, INC., Neenah, Wisconsin 54956 (US)
(72) Inventor: MLEZIVA, Mark, Michael, Whitelaw, WI 54247 (US); ROSLANSKY, Apiromraj, Srisopark, Little Chute, WI 54140 (US); SIEBERS, Bruce, Michael, Kimberly, WI 54136-2302 (US)
(74) Representative: DIEHL GLAESER HILTL & PARTNER
(86) International application number: US9516175
(87) International publication number: WO9619174

(56) References cited:
- US-A- 5 312 387

## Description

The present invention relates to a disposable absorbent product comprising an improved fastening tab. Specifically, the present invention relates to a disposable absorbent product such as a diaper which comprises a fastening tab having an improved ability to attach the absorbent product about a wearer.

Disposable absorbent products are known in the art. Such products include diapers, adult incontinent products, and the like and are typically fastened about a wearer's waist through the use of fastening tabs. The majority of commercially available absorbent products employ an adhesive fastening tab for fastening the product about the waist of a wearer. Mechanical fasteners, such as hook-and-loop fasteners, are known in the art for use in connection with fastening tabs to fasten disposable absorbent products about the waist of a wearer. Such mechanical fasteners have not been introduced into widespread commercial use. This is due to a number of reasons. Such mechanical fasteners are typically more expensive than adhesive fasteners. Since the absorbent products are intended to be disposable, such mechanical fasteners have often been prohibitively expensive.

Research efforts have been made toward the goal of reducing the cost of such mechanical fasteners thereby rendering them more suitable for use on the fastening tabs of disposable absorbent products. Lower cost mechanical fasteners have been developed. Unfortunately, in many instances, these low cost mechanical fasteners are not able to provide the level of attachment necessary to securely fasten a disposable absorbent product about the waist of a wearer.

From US-A-5,312,387 a fastening tab for a disposable diaper is known having a free end and a manufacturer's end. The free end has a distal half with a maximum width and a proximal half with a minimum width. The distal end of the attached fastening tab provides a more secure closure and is capable of being cut from a stock roll in a repeating pattern without any waste.

Accordingly, it is desirable to develop fastening tabs for use on disposable absorbent products, which fastening tabs are configured or designed to make use of lower cost mechanical fastening materials while still providing an acceptable level of attachment. It is to this goal that the present invention is directed.

The present invention intends to overcome the above-mentioned problems. The object is solved by the fastening tab according to independent claim 1, and a disposable absorbent product according to independent claim 10.

Further advantages, features, aspects and details of the invention are evident from the dependent claims, the description and the accompanying drawings.

In a first aspect, the present invention is directed to a fastening tape. The fastening tape comprises a manufacturer's bond end which is attached to a disposable absorbent product and a user's end. The user's end is adapted to secure the disposable absorbent product on a wearer. The user's end of the fastening tab comprises a mechanical fastener component and is configured to have a disengagement ratio of at least 2:1.

The disengagement ratio is achieved by placing at least a portion of said fastening tab in shear when said fastening tab is subjected to peel forces wherein said user's end (26) is multi-lobed, said lobes (42, 44, 46) defining a valley (48) in said mechanical fastener component (32; 40; 64; 76) of at least 5 millimeters and an angle in said mechanical fastener component of from about 0° to about 60°, and/or wherein said fastening tab (22; 36; 58; 72) defines shear channels (66) in said mechanical fastener component (32; 40; 64; 76), and/or wherein said mechanical fastener component (32; 40; 64; 76) defines an unattached edge (80) of at least about 2 millimeters in length and/or wherein said user's end is multi-lobed, said lobes defining one or more valleys (48), said valleys (48) being configured to possess a valley floor (54) which is generally perpendicular to a longitudinal centerline (52) of the fastening tab.

In another aspect, the present invention relates to a disposable absorbent product. The disposable absorbent product comprises an outer cover, a bodyside liner, and an absorbent core located between the bodyside liner and the outer cover. The disposable absorbent product further comprises a fastening tab according to the above description.

In a particular aspect, the present invention relates to a fastening tab comprising a manufacturer's bond end which is attached to a disposable absorbent product and a user's end. The user's end is adapted to secure the disposable absorbent product on a wearer. The user's end comprises a mechanical fastener component and is multi-lobed. The lobes define a valley in said mechanical fastener component of at least 5 millimeters and an angle in said mechanical fastener component of from about 0° to about 60°.

In a further particular aspect, the present invention is directed to a fastening tab which comprises a manufacturer's bond end which is attached to a disposable absorbent product and a user's end. The user's end is adapted to secure the disposable absorbent product on a wearer. The user's end comprises a mechanical fastener component and is cut to define a shear channel in said mechanical fastener component.

The invention will be better understood by reference to the following description of embodiments of the invention taken in conjunction with the accompanying drawings, wherein:
Fig. 1 illustrates a top plan view of a partially cutaway diaper according to the present invention.
Fig. 2 illustrates a cross-sectional view taken along line 2-2 of Fig. 1.
Fig. 3 illustrates one embodiment of a fastening tab according to the present invention.
Fig. 4 illustrates an alternate embodiment of a fastening tab according to the present invention.
Fig. 5 illustrates an alternate embodiment of a fastening tab according to the present invention.
Fig. 6 illustrates an alternate embodiment of a fastening tab according to the present invention.
Fig. 7 illustrates an alternate embodiment of a fastening tab according to the present invention engaged with a loop material.
Fig. 8 illustrates the fastening tab illustrated in Fig. 7 during disengagement of the hook material present on the fastening tab from the loop material.
Fig. 9 illustrates an alternate embodiment of a fastening tab according to the present invention engaged with a loop material.
Fig. 10 illustrates a cross-sectional view of the fastening tab illustrated in Fig. 9 taken along line 10-10 of Fig. 9.
Fig. 11 illustrates an alternate embodiment of a fastening tab according to the present invention.
Fig. 12 illustrates an alternate embodiment of a fastening tab according to the present invention.
Fig. 13 illustrates an alternate embodiment of a fastening tab according to the present invention.

In one aspect, the present invention relates to a fastening tab for use on a disposable absorbent product such as a diaper or adult incontinent product. The description which follows is in connection with a disposable absorbent infant diaper. Nonetheless, it is to be understood that the invention is equally applicable to other disposable absorbent products.

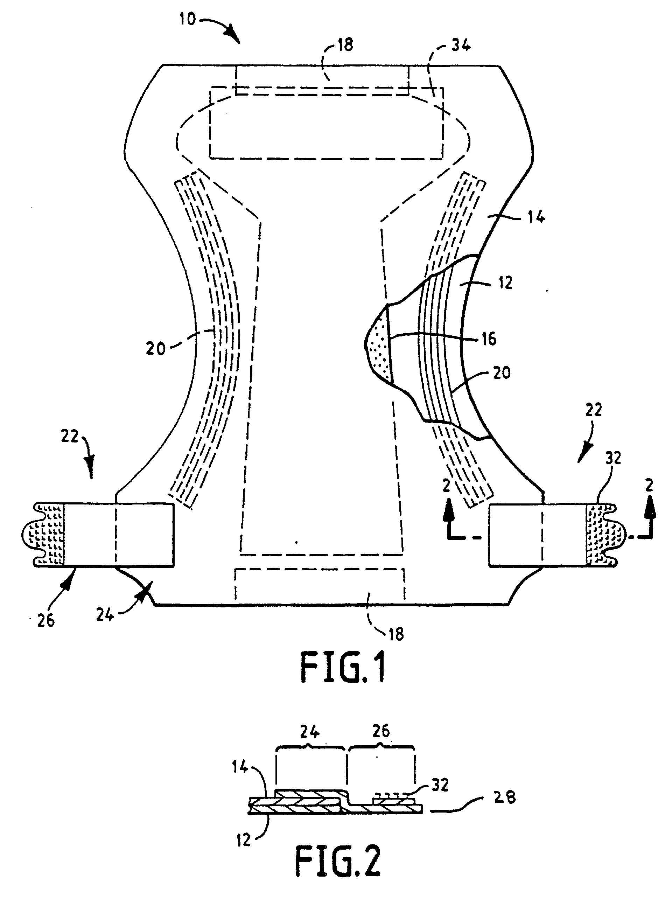

The present invention can best be understood by reference to the figures in which Fig. 1 illustrates a top plan view, partially cutaway, of an infant diaper according to the present invention. Diaper 10 comprises an outer cover 12, a bodyside liner 14, and an absorbent core 16 located between the outer cover 12 and the bodyside liner 14. The diaper 10 may optionally contain waist elastics 18 and leg elastics 20. The diaper 10 also comprises fastening tabs 22. The fastening tabs 22 comprise a manufacturer's bond end 24 attached to diaper 10 and a user's end 26 adapted to secure the diaper about the waist of a wearer. Suitable diaper components and configurations are shown, for example, in U.S. Patents 4,798,603 issued January 17, 1989, to Meyer et al.; U.S. 5,176,668 issued January 5, 1993, to Bernardin; U.S. 5,176,672 issued January 5, 1993, to Bruemmer et al.; U.S. 5,192,606 issued March 9, 1993, to Proxmire et al.; US 5,509,915 (U.S. Patent Application Serial No. 08/096,654 filed July 22, 1993, in the name of Hanson et al.) and US 5,660,666 and US 5,656,111 (U.S. Patent Application Serial No. 08/263,281 filed June 21, 1994, in the name of Dilnik et al).

As used herein, reference to a manufacturer's bond end is intended to refer to that portion of a fastening tab which is attached to the diaper by the manufacturer of the diaper as part of the diaper production process. That is, the manufacturer's end is generally intended to be permanently attached to the diaper.

As used herein, reference to a user's end is intended to refer to that portion of the fastening tab which is used by the consumer to fasten the diaper about the waist of an infant. The user's end of the fastening tab is generally designed to be refastenable such that the diaper can be fastened and refastened about a wearer through the use of the user end of the fastening tab. Thus, the attachment formed by the user end of the fastening tab is generally nonpermanent.

Methods of bonding the fastening tab 22 to the diaper 10 to define the manufacturer's end are known to those skilled in the art. For example, the manufacturer's bond end can be formed by adhesive bonding, sonic bonding, a combination of adhesive and sonic bonding, thermal bonding, and the like. As discussed above, the method of attachment used to form the manufacturer's bond end is generally intended to be permanent. As can be seen from reference to Fig. 2, in the illustrated embodiment, the fastening tab is formed from a first substrate 28. The first substrate is bonded to bodyside liner 14 in the area which forms the manufacturer's bond end 24 of the fastening tab. This bonding is generally permanent and forms the manufacturer's bond end 24 of the fastening tab 22.

The user's end 26 of the fastening tab 22 comprises a mechanical fastener component such as the hook material 32 illustrated in Figs. 1 and 2. As used herein, reference to a mechanical .fastener component is intended to refer to a material which is capable of mechanically engaging with a second mechanical fastener component. Examples of such mechanical fastener components include hook or hooklike materials (mushrooms and the like) in combination with loop or looplike materials. It is also possible for the mechanical fastener components to have similar constructions which are capable of engaging with one another. In the embodiment illustrated in Figs. 1 and 2, the hook material 32 is adapted to engage with a loop material 34 located on the longitudinal end of diaper 10 opposite fastening tabs 22.

A wide variety of hook or hooklike materials are known to those skilled in the art. Suitable hook materials include those available from Velcro® U.S.A. under the trade designation CFM-15; CFM-22-1097; CFM-22-1121; CFM-22-1162; CFM-25-1003; and CFM-29-1003, as well as those available from the Minnesota Mining and Manufacturing Company, St. Paul, Minnesota, under the trade designation CS-200. Similarly, a wide variety of loop or looplike materials are known to those skilled in the art. Suitable loop materials include woven or knitted loops, such as those commercially available from Guilford Mills under the trade designation 34285, as well as nonwoven loop materials, such as those formed by spunbond, meltblown, or carding processes. Suitable nonwoven loop materials are disclosed, for example, in U.S. Patents 4,720,415 issued January 19, 1988, in the name of Vander Wielen et al.; 5,336,545 issued August 9, 1994, in the name of Morman; 5,226,992 issued July 13, 1993, in the name of Morman; in 4,663,220 issued May 5, 1987, in the name of Wisneski et al.; and in US 5,882,769 (pending U.S. Patent Application Serial No. 07/997,800 filed December 29, 1992, in the name of McCormack et al). In the illustrated embodiment, hook material 32 is illustrated as being located on fastening tab 22, while the loop material 34 is located on the outer cover 12 of diaper 10. Alternatively, the loop material may comprise the outer cover 12 of diaper 10. Thus, the outer cover 12 may comprise a material such as that described in US 5,882,769 (U.S.S.N. 07/997,800) referred to above. Of course, it is possible to reverse the location of the hook-and-loop materials so that the loop material is present on the fastening tabs 22 and the hook material is present on the outer cover of the diaper 10.

The fastening tab 22 is configured to provide a disengagement ratio of at least 2:1. As used herein, the disengagement ratio is intended to refer to the ratio of the disengagement strength of the fastening tab design (in grams. per linear mm (inch)) to the disengagement strength (in grams per linear mm (inch)) of a rectangular fastening tab formed from the identical materials but being rectangular in shape. The method by which the disengagement ratio is determined is set forth in greater detail below in connection with the examples.

Many mechanical fastener components possess sufficient shear strength to be commercially viable for use on disposable absorbent products. As used herein, reference to shear strength refers to the force required to separate the mechanical fastener components (such as a hook-and-loop material) when the mechanical fastener components are subjected to opposing forces in the plane of attachment of the components.

Conversely, many mechanical fastener components possess insufficient peel strength to allow for successful commercial use of such materials on disposable absorbent products. As used herein, reference to peel strength refers to the force required to separate the mechanical fastener components (such as a hook-and-loop material) when one component is subjected to a peel force.

Applicants have sought to design fastening tabs which possess improved peel strength. Specifically, Applicants have sought to modify the design of the fastener tabs such that, during peel mode separation, at least a portion of the fastener tab is placed into a shear mode prior to separation of the components. That is, since certain mechanical fastening tabs tend to disengage more easily (with less force) in peel mode more than in shear mode, Applicants have sought to design fastening tabs having a design which, when subjected to peel mode forces, results in the mechanical fastener components being placed into a shear mode prior to separation.

Typical fastening tabs employ a generally rectangular or square piece of mechanical fastener component attached across generally its entire surface to the substrate from which the fastening tab is formed. By altering the configuration of the mechanical fastener components present on the fastening tabs, Applicants can increase the disengagement strength of the fastening tab. This increase in peel strength is quantified as a disengagement ratio as will be described in greater detail below in connection with the examples.

Thus, the user's end of the fastening tabs of the present invention are configured to have a disengagement ratio of at least 2:1, alternatively of at least 5:1, alternatively of at least about 10:1, and still further alternatively of at least about 15:1.

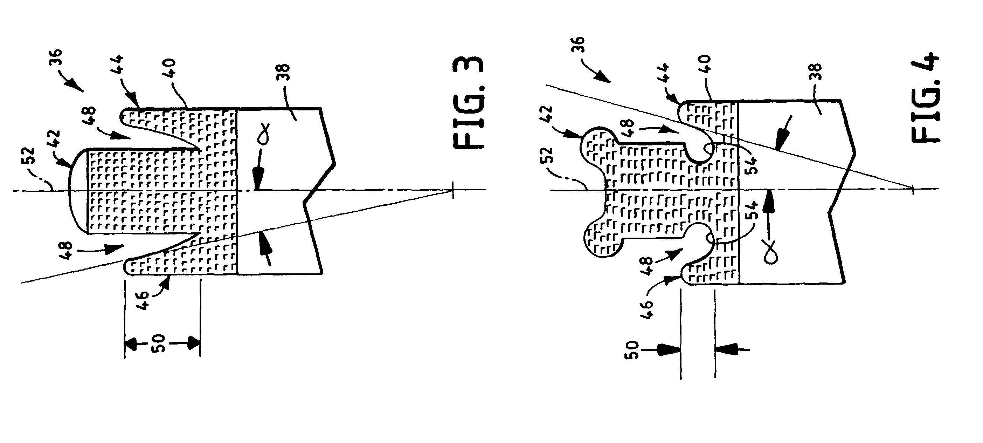

Figs. 3-13 illustrate specific fastening tabs according to the present invention. Figs. 3-6 illustrate multi-lobed fastening tabs. Fig. 3 illustrates a user's end of fastening tab 36. The user's end of fastening tab 36 comprises a first substrate 38 to which a mechanical fastener component such as hook material 40 is attached. The fastening tab 36 defines a main, central lobe 42 and side lobes 44 and 46. The configuration of the central lobe 42 and side lobes 44 and 46 serve to define valleys 48 in the mechanical fastener component (hook 40).

Applicants have discovered that formation of valleys 48 in the mechanical fastener components such as hook 40 can result in producing a disengagement ratio of at least about 1.5:1 when the valley in the mechanical fastener component has a depth 50 of at least about 5 millimeters, alternatively of at least about 8 millimeters, alternatively of at least about 15 millimeters. Further, the side lobes 44 and 46 suitably define an angle alpha in said mechanical fastener component 40 with respect to centerline 52 and the inside surface of said side lobes of from about 0° to about 60°. Alternatively, the side lobes 44 and 46 define an angle in said mechanical fastener component of from about 10° to about 55°. By configuring the mechanical fastener component 40 to define the valley and angle alpha discussed above, removal of the mechanical fastener component 40 from a mating mechanical fastener component by removing central lobe 42 in a peel mode has been found to place side lobes 44 and 46 into a shear mode despite the application of peel mode forces. As discussed above, this has been found to produce a mechanical fastening tab having a disengagement ratio of 2:1 or greater. Forming an angle alpha greater, than about 10° has been found to assist in separation of the mechanical fastener components.

Fig. 4 illustrates an alternative embodiment of a fastening tab according to the present invention. In Fig. 4, like components of the fastening tab illustrated in Fig. 3 have like numerals. Again, as illustrated in Fig. 4, the presence of valleys 48 and the angle alpha defined in the mechanical fastener component have been found to produce a fastening tab having a disengagement ratio of 2 or greater. The valleys 48 illustrated in Fig. 4 possess a valley floor 54 which is generally perpendicular to centerline 52. The presence of the valley floor 54, which is generally perpendicular to centerline 52, has been found to increase the disengagement ratio compared to a similar embodiment such as that illustrated in Fig. 3 where no valley floor 54 is defined.

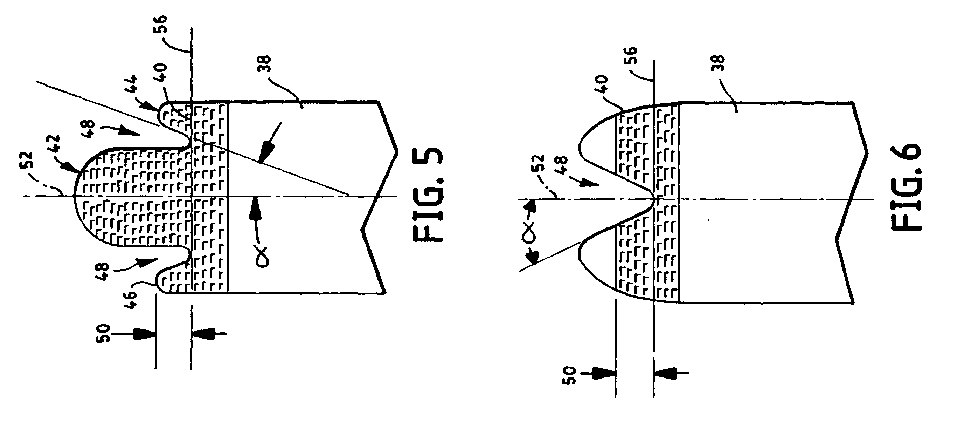

Fig. 5 illustrates a multi-lobed fastening tab such as that illustrated in Figs. 3 and 4. The fastening tab of Fig. 5 defines three lobes (trilobal), central lobe 42, and side lobes 44 and 46. Again, the numerals appearing in Fig. 5 refer to like elements as described above in connection with Figs. 3 and 4. The angle alpha defined by Fig. 5 is approximately 20 degrees, while the angle alpha defined by Fig. 3 is about 10 degrees.

In Figs. 3, 4, and 5, each of the central lobe 42, side lobe 44 and side lobe 46 define a surface area occupied by said mechanical fastener component 40. For the purposes of this application, the surface area of a lobe will refer to that portion of said lobe covered by said mechanical fastener component, with said lobe being defined by a line which connects the lowest point of valley 48 with the nearest adjacent valley; or, in the case of a fastening tab comprising a single valley 48, by a line tangent to the lowest point defined by valley 48 and perpendicular to centerline 52. Thus, with respect to Fig. 5, line 56 serves to define the surface area of central lobe 42 and side lobes 44 and 46 which comprise the surface area of said lobes. Specifically, the area of lobes 42, 44, and 46 covered by mechanical fastener component 40 and located on the side of line 56 most remote from the manufacturer's bond end of the fastening tab constitutes the surface area of the lobes. In the fastening tabs illustrated in Figs. 3, 4, and 5, the central lobe 42 has a surface area which is at least about 50 percent greater than the surface area of at least one of the side lobes 44 or 46. As the fastening tabs illustrated in Figs. 3-5 are generally symmetrical about centerline 52, the surface area of central lobe 42 is at least about 100 percent greater, alternatively about 200 percent greater than the surface area of both the side lobes 44 and 46.

Fig. 6 illustrates a bilobal fastening tab in which the reference numerals refer to like elements as the same reference numerals appearing in Figs. 3-5.

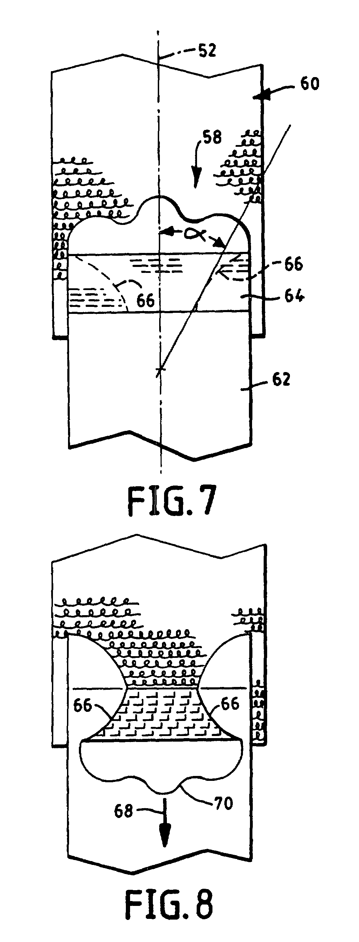

Fig. 7 illustrates an alternative form of fastening tab according to the present invention. With reference to Fig. 7, fastening tab 58 is illustrated in an engaged relationship with loop material 60. Fastening tab 58 includes a first substrate 62 to which a hook material 64 is attached. The hook material 64 engages with the loops of loop material 60. The fastening tab 58 defines shear channels 66 formed by cutting completely through fastening tab 58. The shear channels 66 form an angle alpha relative to centerline 52 of fastening tab 58. Angle alpha is suitably from about 0° to about 60°, alternatively of from about 15° to about 55°.

Fig. 8 illustrates the fastening tab 58 of Fig. 7 being subjected to a peel force in the direction of arrow 68. As can be seen from Fig. 8, as the tip 70 of fastening tab 58 is pulled from an engaged relationship with loop material 60, shear channels 66 allow the hook material 64 to separate such that the hook material located between shear channels 66 is subjected to a peel force and the hook material located on the outside (relative to centerline 52) of shear channels 66 is subjected to both peel forces and to shear forces before separation from the loop material 60. This results in the fastening tab 58 having a disengagement ratio of greater than 2:1.

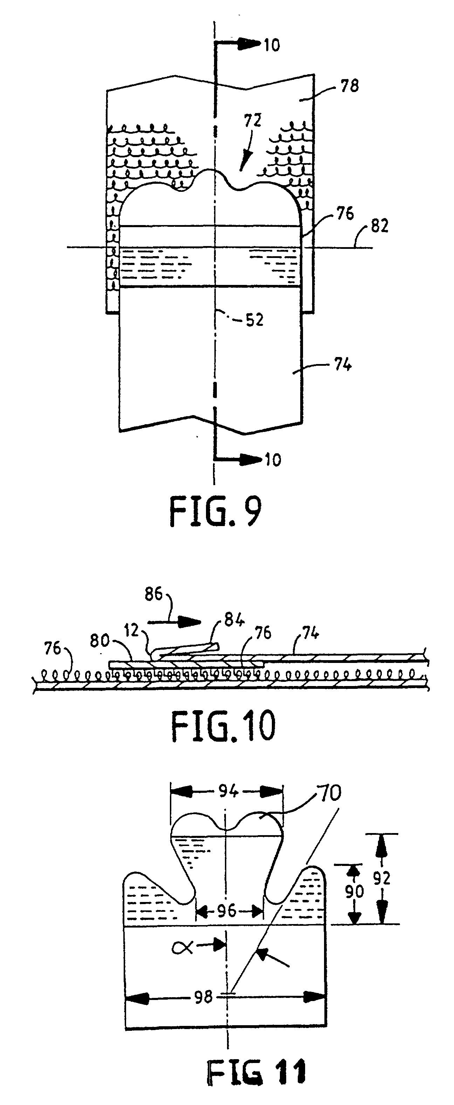

Fig. 9 illustrates an alternative embodiment of a fastening tab according to the present invention. In Fig. 9, fastening tab 72 comprises a first substrate 74 on which is attached a hook material 76. The hook material 76 is shown in an engaged relationship with loop material 78. The hook material 76 is attached to the substrate 74 such that an area along the width (direction perpendicular to centerline 52) is unattached to the first substrate, thus forming unattached edge 80 of hook material 76. The unattached edge 80 is defined by line 82 such that the area of hook material 76 located on the side of line 82 remote from the manufacturer's bond end of the fastening tab 72 is unattached to the first substrate 74. This can best be seen by reference to Fig. 10 which is a cross-sectional view taken along line 10-10 of Fig. 9.

Thus, when edge 84 of the first substrate 74 is subjected to peel forces as indicated by arrow 86, the unattached edge 80 of hook material 76 remains engaged with the loop material 78 until separation of the first substrate 74 from the hook material 76 reaches line 82. At that point, the unattached edge 80 of hook material 76 will be subjected to both shear forces and peel forces. It is desired that the unattached edge of hook material 76 has a length in a direction parallel to centerline 52 of at least about 2 millimeters, preferably of at least about 6 millimeters, preferably of from about 2 to about 10 millimeters. Such a configuration has been found to produce a fastening tab having a disengagement ratio of 2:1 or greater.

Fig. 11 represents another embodiment of fastening tabs according to the present invention. The central lobe 42 includes tips 70 on its top end. The top end has a width 94 and the base of said central lobe 42 has a width 96. The side lobes 44 and 46 have a length 90. The overall length of fastening material along center line 52 is indicated by 92. Further, the side lobes 44 and 46 suitably define an angle alpha with respect to center line 52 and the inside surface of said side lobes.

Alpha defines an angle of from 0° to about 60°, preferably of from about 10° to about 55°.

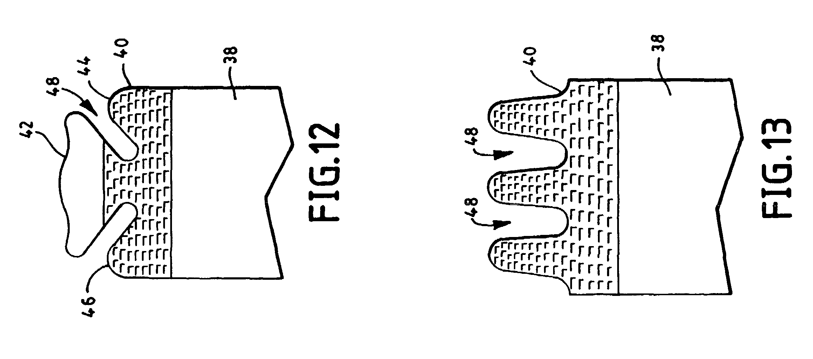

Figs. 12 and 13 represent alternate embodiments of fastening tabs according to the present invention. Again, like components of the fastening tabs described above have like numerals in Figs. 12 and 13.

Designs such as those illustrated in Figs. 3, 4, 5, 11, et seq., provide the advantage that a caregiver can grasp a single lobe or tab to remove the fastening tape. This makes use of the fastening tabs easier from the caregiver's perspective. The center lobe is most generally grasped to accomplish removal.

Many of the designs illustrated in the figures have the advantage that they can be produced in a "nested" configuration, thereby reducing waste associated with production and lowering the costs of such fastening tabs.

### Test Procedures

### Disengagement Ratio

A fastening tab to be tested is provided. The fastening tab comprises a first mechanical fastener component. The first mechanical fastener component of the fastening tab (typically the hook material) should have a maximum width of about 5.08 cm (2 inches) and a maximum length of about 2.54 cm (1 inch). A sample of the second mechanical fastener component with which the first mechanical fastener component is intended to engage is also provided. The sample of the second mechanical fastener component (typically the loop material) is at least about 0.635 cm (0.25 inch) wider and at least about 15.24 cm (6 inches) longer than the fastening tab being tested.

### The following equipment is employed:

A Sintech System 2 tensile tester having a computerized data acquisition system such as a Sintech QAD System commercially available from Sintech, Inc., P.O. Box 14226, Research Triangle Park, North Carolina 27709-4226. The tensile tester is equipped with a computerized data acquisition system capable of determining the peak load (the maximum load, measured in grams, achieved between the designated start and end measurements) and the three peak load (the average of the three highest peak load values, measured in grams, between designated start and end measurements). To determine the three peak load, the computer initiates a peak search after determining a load value which is 5 percent greater than a first minimum value. During a peak search, the computer searches for the maximum load value preceding a 5 percent decrease in load from said maximum load value. This is identified as a peak. The computer then searches for the minimum value preceding a 5 percent increase in load from said minimum value and identifies this value as the new minimum load value. A 5 percent increase in load over this new minimum load initiates another peak search. This process continues until reaching the end measurement.

A 2.04 kg (4.5 pound) rubber-covered handheld roller commercially available from Chemsultants International, Mentor, Ohio 44061-1118 under the designation "Item 6.2."

All testing is conducted in a standard laboratory atmosphere of 23°C ± 2°C and 50 percent ± 5 percent relative humidity.

### Test Process

The second mechanical fastener component (typically a loop material) is securely mounted on a hard flat surface, for example, by clipping one end of the material in the clip of a clipboard, smoothing the material along the surface of the clipboard, and clamping the other end of the material to the clipboard with a clamp such as that commercially available from Publix Supplies under the trade designation IDL Model No. 11240. The first mechanical fastener component of the fastening tab to be tested is then applied to the second mechanical fastener component such that the first mechanical fastener component present on the fastening tab is fully engaged with the second mechanical fastener component in a manner similar to that in which the two would be engaged during the intended use of the absorbent product on which the fastening tab is to be used. The mechanical fastener components are engaged by rolling the 2.04 kg (4.5 pound) roller across the mechanical fastener components and back again. Care is taken such that the only weight applied to the mechanical fastener components is that of the 2.04 kg (4.5 pound) roller with no added weight coming from the handle of the roller or the individual performing the test. The 2.04 kg (4.5 pound) roller is rolled across the test specimen in the width dimension of the first mechanical fastener component.

The engaged fastening tab is then released from its mounting on a surface. The tensile tester has the following test parameters:

| | |
|---|---|
| Crosshead Speed | 500 millimeters per minute (20 inches per minute) |
| Gauge Length | 125 millimeters (5 inches) |
| Load Units | Grams |
| Full Scale Load | 5,000 grams (set the load range so the values fall between 20 and 80 percent of the full-scale load) |
| Start Measurement | 2.54 mm (0.10 inch) |
| End Measurement | 63.5 mm (2.5 inches) |
| Break Sensitivity | 110 percent |
| Peak Criterion | 5 percent |
| Extension Limit High | 69.85 mm (2.75 inches) |

Both the peak load and the three peak average peel values must be taken between the start measurement and the end measurement.

Start and end measurement values are dependent on the size of the area in which the first and second mechanical fastener components are in contact with one another. The values recited above were chosen for a 12.7 mm (1/2 inch) wide hook material (first mechanical fastening component).

During the test, the fastening tab is peeled from the material with which it is engaged in the direction in which the fastening tab would normally be removed during its intended use. The end of the user's end portion of the fastening tab, opposite the manufacturer's bond end, is clamped in the upper jaw of the tensile tester. If the fastening tab is bilobal, only one lobe is clamped in the upper jaw. If the fastening tab is trilobal, the center or longest lobe is clamped in the jaw. The second mechanical fastener component (loop material) extending beyond the user's end of the fastening tab in a direction away from the manufacturer's end of the fastener's tab is clamped in the lower jaw of the tensile tester. The crosshead is started in motion. The data acquisition system records the peak load and the average of the three highest peaks. Six identical samples are tested and the average of the three peak averages for the six samples is determined. This average is normalized by calculating the grams per linear inch based on the width of the first mechanical fastening component present on the fastening tab to give a test value.

To determine the disengagement ratio, the above testing is repeated with a control composite employing the same second mechanical fastener component as in the above test procedure. The same first mechanical fastener component is also tested with the exception that the first mechanical fastening component is in the form of a rectangle 44.45 mm (1.75 inches) wide and 12.7 mm (1/2 inch) long. The rectangle of the first mechanical fastener component is adhesively and ultrasonically secured to a nonwoven substrate such that the entire surface of the first mechanical fastening component is adhered to the substrate and such that 12 millimeters of the substrate extends beyond one edge of the first mechanical fastening component, and so that at least 50 millimeters of the substrate extends beyond the other edge of the mechanical fastening component. In this way, a fastening tab is formed. A suitable adhesive is commercially available from Findley Adhesives under the designation H-2096. A suitable nonwoven substrate is a neck-bonded-laminate (NBL) such as those described in U.S. Patent No. 5,226,992 issued July 13, 1993, to Morman. The NBL employed included three layers. The outer two layers were polypropylene spunbond layers having a basis weight of 24.1 g (0.85 ounce) per 0.84 m² (square yard) and being formed from 2-3 denier fibers. The center layer was an elastomeric film having a thickness of 0.0381 mm-0.0508 mm (0.0015-0.002 inch) and being formed from Kraton^{TM} G2755, a polymeric resin available from Shell Chemical Company.

The first and second mechanical fastener components are then engaged as described above through the use of the 2.04 kg (4.5 pound) roller. The peel strength of the rectangular mechanical fastener component is then determined as described above. This test is repeated six times, using new materials for each test, to determine an n = 6 three peak average peel strength. This number is then normalized by dividing by the 44.45 mm (1.75 inch) width of the first mechanical fastener component to give a control value.

The test value and control value are then used to calculate the disengagement ratio according to the formula:

### Test Value

### Control Value

This test is similar to American Society for Testing and Materials (ASTM) Test Designation D5170-91 "Peel Strength ("T"-Method of Hook-and-Loop Touch Fasteners)".

### Examples

In the following examples, a hook material available from the Minnesota Mining and Manufacturing Company (3M), St. Paul, Minnesota, under the designation CS-200 hook is employed. The loop material employed is a knit loop material commercially available from Guilford Mills, Inc., under the trade designation 34285 loop. Six different fastening tabs are designed using the above materials. For each of the fastening tabs, the hook material is adhesively and ultrasonically (about 6.25 percent bond area) attached to an NBL material having a basis weight of about 0.116 kg per 0.836 m² (4.1 ounce per square yard) (described above in the Test Procedures section). The adhesive employed is provided on the CS-200 material as received from 3M. If the CS-200 is not provided with an adhesive backing, 3M double-sided tape (948y) can be used. The following fastening tabs are prepared:

Fastening Tab 1 - This tab has the configuration generally illustrated in Fig. 11 in which dimension 90 is 13 millimeters, dimension 92 is 20 millimeters, dimension 94 is 23 millimeters, dimension 96 is 17 millimeters, dimension 98 is 45 millimeters, and angle alpha is about 45 degrees.

Fastening Tab 2 - A fastening tab is prepared having the general configuration illustrated in Figs. 7 and 8 wherein the width of the hook material 64 (in a direction generally perpendicular to centerline 52) is 45 millimeters, the length of the hook material 64 (in a direction generally parallel to centerline 52) is 13 millimeters, and the shear channel 66 defines an angle alpha of 30 degrees. The distance between tip 70 and the adjacent edge of hook material 64 is about 13 millimeters.

Fastening Tab 3 - A fastening tab is prepared having the general configuration illustrated in Fig. 6 wherein the hook material 40 has a width of about 45 millimeters, a length of about 13 millimeters, and defines a valley 48 having a depth 50 of 5 millimeters. The fastening tab defines an angle alpha of about 20 degrees. The ends of the lobes not covered by the hook material 40 have a length of about 13 millimeters.

For Fastening tabs 4 and 5, the hook material is adhesively attached to 28.4 g per 0.836 m² (1.0 ounce per square yard) spunbond material which is then adhesively attached to the NBL material. The hook material, spunbond material and NBL material are then ultrasonically bonded together (about 6.25 percent bond area).

Fastening Tab 4 - A fastening tab is prepared having the general configuration illustrated in Figs. 9 and 10. The hook material 76 has a width of 45 millimeters, a length of 13 millimeters, and defines an unattached edge (80) having a length of 10 millimeters. The distance between edge 84 and line 82 is 23 millimeters.

Fastening Tab 5 - A fastening tab is prepared identical to fastening tab 4 with the exception that the unbonded edge 80 is 5 millimeters in length and the distance between edge 84 and line 82 is 18 millimeters.

Control Fastening Tab - A fastening tab is prepared from the same materials described above. The hook material has a width of 44.45 mm (1.75 inches) and a length of 12.7 mm (0.5 inch). The hook material is adhesively attached to 28.4 g per 0.836 m² (1.0 ounce per square yard) spunbond material which is then adhesively attached to a rectangular piece of the NBL material described above having a width of 44.45 mm (1.75 inches) and a length of 85 millimeters. The hook material, spunbond material and NBL material are then ultrasonically bonded together (about 6.25 percent bond area). Thirteen millimeters of the NBL material extend beyond one edge of the hook material to define a finger tab. The hook material is adhesively secured to the NBL material across its entire surface.

The above fastening tabs are subjected to the testing described above to determine their disengagement ratios (individual fastening tabs 1-5 compared to control fastening tabs). The results of this testing are set forth in Table 1.

**TABLE 1***

| Fastening Tab | 3 peak Avg. Peel (g) | Standard Deviation | Normalized Peel¹ | | Ratio² |
|---|---|---|---|---|---|
| 1 | 1389.3 | 413.2 | 31.26 | (793.9) | 8.4 |
| 2 | 1602.4 | 207.4 | 36.05 | (915.7) | 9.6 |
| 3 | 580.6 | 295.3 | 13.06 | (331.8) | 3.5 |
| 4 | 3008.8 | 412.6 | 67.69 | (1719.3) | 18.1 |
| 5 | 2087.8 | 212.4 | 46.97 | (1193.0) | 12.6 |
| Control | 166.3 | 73.1 | 3.74 | (95) | 1 |

| | | | | | |
|---|---|---|---|---|---|
| * Testing was conducted at ambient temperature and humidity levels (^{~}23°C; <50% relative humidity) ¹ In grams per mm (grams per linear inch) | | | | | |
| ² Disengagement ratio | | | | | |

As can be seen from the above data, the specific fastening tab designs described herein have a disengagement ratio greater than 2:1.

Having thus described the invention in full detail, it will be readily apparent to a person of ordinary skill in the art that various changes and modifications can be made without departing from the scope of the invention. All of the changes and modifications are contemplated as being within the scope of the present invention as defined by the following claims.

## Claims

1. A fastening tab (22; 36; 58; 72), said fastening tab (22; 36; 58; 72) comprising:
a manufacturer's bond end (24) attached to a disposable absorbent product (10); and
a user's end (26) adapted to secure said disposable absorbent product (10) on a wearer, said user's end (26) comprising a mechanical fastener component (32; 40; 64; 76) and **characterized in that** it is configured to have a disengagement ratio of at least 2:1, wherein said disengagement ratio is achieved by placing at least a portion of said fastening tab in shear when said fastening tab is subjected to peel forces wherein said user's end (26) is multi-lobed, said lobes (42, 44, 46) defining a valley (48) in said mechanical fastener component (32; 40; 64; 76) of at least 5 millimeters and an angle in said mechanical fastener component of from about 0° to about 60°, and/or wherein said fastening tab (22; 36; 58; 72) defines shear channels (66) in said mechanical fastener component (32; 40; 64; 76), and/or wherein said mechanical fastener component (32; 40; 64; 76) defines an unattached edge (80) of at least about 2 millimeters in length and/or wherein said user's end is multi-lobed, said lobes defining one or more valleys (48), said valleys (48) being configured to possess a valley floor (54) which is generally perpendicular to a longitudinal centerline (52) of the fastening tab.

2. The fastening tab according to claim 1 wherein said user's end (26) has a disengagement ratio of at least 5:1.

3. The fastening tab according to claim 1 wherein said user's end (26) has a disengagement ratio of at least about 10:1.

4. A fastening tab according to claim 1 wherein said user's end (26) has a disengagement ratio of at least about 15:1.

5. The fastening tab according to at least one of the preceding claims wherein said mechanical fastener component (32; 40; 64; 76) is the hook component of a hook-and-loop fastener.

6. A fastening tab according to claim 1 wherein said user's end is multilobed and wherein said lobes (42, 44, 46) define a valley (48) in said mechanical fastener component (32; 40; 64; 76) of at least about 8 millimeters.

7. The fastening tab according to at least one of claims 1 or 6 wherein said user's end is multilobed and wherein said lobes (42, 44, 46) define an angle in said mechanical fastener component (32; 40; 64; 76) of from about 10° to about 55°.

8. The fastening tab according to at least one of the preceding claims wherein said user's end (26) is multilobed, defining three lobes (42, 44, 46), each said lobe (42; 44; 46) having a surface area, said surface area of one of said lobes (42; 44; 46) being at least 50 percent greater than at least one of the remaining lobes.

9. The fastening tab according to at least one of claims 1 to 5 wherein said fastening tab (22; 36; 58; 72) defines two shear channels (66).

10. A disposable absorbent product, said product comprising:
an outer cover (12);
a bodyside liner (14);
an absorbent core (16) located between said bodyside liner (14) and said outer cover (12); and
a fastening tab according to at least one of the preceding claims.

## Patentansprüche

1. Befestigungssystem (22; 36; 58; 72), wobei das Befestigungssystem (22; 36; 58; 72) umfasst:
ein Verbundende (24) des Herstellers, das an einem absorbierenden Wegwerfprodukt (10) angebracht ist; und
ein Ende (26) für den Verwender, das vorgesehen ist, um ein absorbierendes Wegwerfprodukt (10) an einem Träger zu halten, wobei das Ende (26) für den Verwender eine mechanische Befestigungskomponente (32; 40; 64; 76) umfasst und **dadurch gekennzeichnet, dass** es gestaltet ist, um ein Loslöseverhältnis von wenigstens 2:1 aufzuweisen, wobei das Loslöseverhältnis durch Anordnen wenigstens eines Bereichs des Befestigungssystems in Scherung, wenn das Befestigungssystem Abziehkräften ausgesetzt ist, wobei das Ende (26) für den Verwender mehrflüglig ist, wobei die Flügel (42, 44, 46) in der mechanischen Befestigungskomponente (32; 40; 64; 76) eine Kerbe (48) von wenigstens 5 Millimetern und in der mechanischen Befestigungskomponente einen Winkel von etwa 0° bis etwa 60° beschreiben, und/oder wobei das Befestigungssystem (22; 36; 58; 72) Scherkanäle (66) in der mechanischen Befestigungskomponente (32; 40; 64; 76) beschreibt, und/oder wobei die mechanische Befestigungskomponente (32; 40; 64; 76) eine unbefestigte Kante (80) beschreibt, die wenigstens etwa 2 Millimeter lang ist, und/oder wobei das Ende für den Verwender mehrflüglig ist, wobei die Flügel eine oder mehrere Kerben (48) beschreiben, wobei die Kerben (48) geeignet sind, einen Kerbenboden (54) aufzuweisen, der im Allgemeinen senkrecht zu einer längsverlaufenden Mittellinie (52) des Befestigungssystems verläuft.

2. Befestigungssystem gemäß Anspruch 1, wobei das Ende (26) für den Verwender ein Loslöseverhältnis von wenigstens 5:1 aufweist.

3. Befestigungssystem gemäß Anspruch 1, wobei das Ende (26) für den Verwender ein Loslöseverhältnis von wenigstens etwa 10:1 aufweist.

4. Befestigungssystem gemäß Anspruch 1, wobei das Ende (26) für den Verwender ein Loslöseverhältnis von wenigstens etwa 15:1 aufweist.

5. Befestigungssystem gemäß wenigstens einem der vorhergehenden Ansprüche, wobei die mechanische Befestigungskomponente (32; 40; 64; 76) die Hakenkomponente einer Haken- und Schlaufenbefestigung ist.

6. Befestigungssystem gemäß Anspruch 1, wobei das Ende für den Verwender mehrflüglig ist und wobei der Flügel (42, 44, 46) eine Kerbe (48) in der mechanischen Befestigungskomponente (32; 40; 64; 76) beschreibt, die wenigstens etwa 8 Millimeter beträgt.

7. Befestigungssystem gemäß wenigstens einem der Ansprüche 1 oder 6, wobei das Ende für den Verwender mehrflüglig ist und wobei der Flügel (42, 44, 46) einen Winkel in der mechanischen Befestigungskomponente (32; 40; 64; 76) beschreibt, der von etwa 10° bis etwa 55° beträgt.

8. Befestigungssystem gemäß wenigstens einem der vorhergehenden Ansprüche, wobei das Ende (26) für den Verwender mehrflüglig ist und drei Flügel (42, 44, 46) beschreibt, wobei jeder der Flügel (42; 44; 46) einen Oberflächenbereich aufweist, wobei der Oberflächenbereich einer der Flügel (42; 44; 46) um wenigstens 50 Prozent größer ist als wenigstens einer der übrigen Flügel.

9. Befestigungssystem gemäß wenigstens einem der Ansprüche 1 bis 5, wobei das Befestigungssystem (22; 36; 58; 72) zwei Scherkanäle (66) beschreibt.

10. Absorbierendes Wegwerfprodukt, wobei das Produkt umfasst:
eine äußere Abdeckung (12);
eine körperseitige Einlage (14);
einen absorbierenden Kern (16), der zwischen der körperseitigen Einlage (14) und der äußeren Abdeckung (12) angeordnet ist; und
ein Befestigungssystem gemäß wenigstens einem der vorhergehenden Ansprüche.

## Revendications

1. Patte d'attache (22 ; 36 ; 58 ; 72), ladite patte d'attache (22 ; 36 ; 58 ; 72) comprenant :
une extrémité de liaison à la fabrication (24) fixée à un produit absorbant jetable (10) ; et
une extrémité "utilisateur" (26) adaptée à retenir ledit produit absorbant jetable (10) sur un porteur, ladite extrémité "utilisateur" (26) comprenant un composant d'attache mécanique (32 ; 40 ; 64 ; 76) et étant **caractérisée en ce qu'**elle est configurée pour avoir un rapport de séparation d'au moins 2:1, ledit rapport de séparation étant obtenu en soumettant au moins une portion de ladite patte d'attache à un cisaillement lorsque ladite patte d'attache est soumise à des forces de pelage, patte d'attache dans laquelle ladite extrémité "utilisateur" est multilobée, lesdits lobes (42, 44, 46) définissant, dans ledit composant d'attache mécanique (32 ; ; 40 ; 64 ; 76), une échancrure (48) d'au moins 5 mm et un angle compris entre environ 0° et environ 60° et/ou ladite patte d'attache (22 ; 36 ; 58 ; 72) définit des canaux de cisaillement (66) dans ledit composant d'attache mécanique (32 ; 40 ; 64 ; 76) et /ou ledit composant d'attache mécanique (32 ; 40 ; 64 ; 76) définit un bord non fixé (80) d'au moins environ 2 mm de long et/ou ladite extrémité "utilisateur" est multilobée, lesdits lobes définissant une ou plusieurs échancrures (48), lesdites échancrures (48) étant configurées pour présenter un pied d'échancrure (54) qui est généralement perpendiculaire à une ligne centrale longitudinale (52) de la patte d'attache.

2. Patte d'attache selon la revendication 1, dans laquelle ladite extrémité "utilisateur" (26) a un rapport de séparation d'au moins 5:1.

3. Patte d'attache selon la revendication 1, dans laquelle ladite extrémité "utilisateur" (26) a un rapport de séparation d'au moins environ 10:1.

4. Patte d'attache selon la revendication 1, dans laquelle ladite extrémité "utilisateur" (26) a un rapport de séparation d'au moins environ 15:1.

5. Patte d'attache selon l'une au moins des revendications précédentes, dans laquelle ledit composant d'attache mécanique est le composant à crochets (32 ; 40 64 ; 76) d'une attache à crochets et bouclettes.

6. Patte d'attache selon la revendication 7, dans laquelle l'extrémité "utilisateur" est multilobée, lesdits lobes (42, 44, 46) définissant, dans ledit composant d'attache mécanique (32, 40, 64, 76), une échancrure (48) d'au moins environ 8 mm.

7. Patte d'attache selon l'une au moins des revendications 1 à 6, dans laquelle l'extrémité "utilisateur" est multilobée, lesdits lobes (42, 44, 46) définissant, dans ledit composant d'attache mécanique (32, 40, 64, 76), un angle compris entre environ 10° et environ 55°.

8. Patte d'attache selon l'une au moins des revendications précédentes, dans laquelle ladite extrémité "utilisateur" (26) est multilobée, définissant trois lobes (42, 44, 46), chacun desdits lobes (42, 44, 46) ayant une aire superficielle, ladite aire superficielle de l'un desdits lobes (42, 44, 46) étant d'au moins 50% supérieure à celle de l'un au moins desdits lobes restants.

9. Patte d'attache selon l'une au moins des revendications 1 à 5, dans laquelle ladite patte d'attache (22 ; 36 ; 58 ; 72) définit deux canaux de cisaillement (66).

10. Produit absorbant jetable, ledit produit comprenant :
une enveloppe extérieure (12) ;
une doublure côté corporel (14) ;
un noyau absorbant (16) situé entre ladite doublure côté corporel (14) et ladite enveloppe extérieure (12) ; et
une patte d'attache selon l'une au moins des revendications précédentes.
